# EUROPEAN PATENT APPLICATION

(11) **EP 4 541 333 A1**
(43) Date of publication of application: **23.04.2025**
(21) Application number: 24214046.5
(22) Date of filing: 21.09.2021
(51) Int. Cl.: A61F 13/534

(54) **ABSORBENT ARTICLE WITH IMPROVED MULTI-LAYERED CORE**

(30) Priority: 22.09.2020 EP 20197374; 30.11.2020 EP 20210559; 21.01.2021 EP 21152698
(62) Divisional of application: 23206739.7
(71) Applicant: Ontex BV, 9255 Buggenhout (BE); Ontex Group NV, 9320 Erembodegem (BE)
(72) Inventor: Pannwitt, Stefanie, 56727 Mayen (DE); Lambertz, Christina, 56566 Neuwied (DE); Ingenfeld, Björn, 53115 Bonn (DE); Mailinger, Christel, 65604 Elz (DE); Weber, Ainas, 53474 Bad Neuenahr-Ahrweiler (DE); Granado, Martín, 56727 Mayen (DE)
(74) Representative: Macchetta, Andrea

(57) **Abstract**

An absorbent article comprising: a liquid permeable topsheet, a liquid impermeable backsheet, and an absorbent core positioned between said topsheet and backsheet, wherein the absorbent core comprises an absorbent material, said absorbent material comprising a mixture, or blend, of cellulose fibers and superabsorbent polymers, wherein the absorbent core comprises at least a top layer (L1) and a bottom layer (L2) wherein the bottom layer (L2) is positioned between the top layer (L1) and the backsheet, and wherein said absorbent material is contained within at least one core wrap substrate enclosing said absorbent material therein, wherein the at least top layer (L1) and a bottom layer (L2) are directly stacked one on top of the other to form a contact zone (Zc) where the top layer (L1) directly adjoins to the bottom layer (L2), and wherein the top layer (L1) comprises one or more first superabsorbent polymer grades (SAP1) and the bottom layer (L2) comprises one or more second superabsorbent polymer grades (SAP2), wherein the first superabsorbent polymer grades (SAP1) have an AUL that is greater than the AUL of second superabsorbent polymer grades (SAP2), and wherein the first superabsorbent polymer grades (SAP1) have an AUL, as measured according to the test method herein, of greater than 15 g/g.

## Description

### TECHNICAL FIELD

The disclosure relates to absorbent articles such as disposable absorbent articles, preferably selected from the group consisting of diapers (whether for baby or adults), pants (whether for baby or adults), pantiliners, briefs, sanitary napkins, and combinations thereof.

### BACKGROUND

Disposable absorbent garments such as infant diapers or training pants, adult incontinence products and other such products typically were constructed with a moisture-impervious outer backsheet, a moisture-pervious body-contacting topsheet, and a moisture-absorbent core sandwiched between the topsheet and backsheet. Much effort has been made to find cost-effective materials for absorbent cores that display favorable liquid absorbency and retention. Superabsorbent materials in the form of granules, beads, fibers, bits of film, globules, etc., have been favored for such purposes. Such superabsorbent materials generally (also referred to as superabsorbent polymer particles or SAP) are polymeric gelling materials that are capable of absorbing and retaining large quantities of liquid, such as water and body wastes, relative to their own weight, even under moderate pressure.

The superabsorbent material generally is a water-insoluble but water-swellable polymeric substance capable of absorbing water in an amount which is at least ten times the weight of the substance in its dry form. A superabsorbent material comprising a polymer is hereinafter referred to as a superabsorbent polymer or "SAP". In one type of SAP, the particles or fibers may be described chemically as having a back bone of natural or synthetic polymers with hydrophilic groups or polymers containing hydrophilic groups being chemically bonded to the back bone or in intimate admixture therewith. Included in this class of materials are such modified polymers as sodium neutralized cross-linked polyacrylates and polysaccharides including, for example, cellulose and starch and regenerated cellulose which are modified to be carboxylated, phosphonoalkylated, sulphoxylated or phosphorylated, causing the SAP to be highly hydrophilic. Such modified polymers may also be cross-linked to reduce their water-solubility.

The ability of a superabsorbent material to absorb liquid typically is dependent upon the form, position, and/or manner in which particles of the superabsorbent are incorporated into the absorbent core. Whenever a particle of the superabsorbent material and absorbent core is wetted, it swells and forms a gel. Gel formation can block liquid transmission into the interior of the absorbent core, a phenomenon called "gel blocking." Gel blocking prevents liquid from rapidly diffusing or wicking past the "blocking" particles (e.g., those particles that have swelled and touched an adjacent swelled particle), causing portions of a partially hydrated core to become inaccessible to multiple doses of urine. Further absorption of liquid by the absorbent core must then take place via a diffusion process. This is typically much slower than the rate at which liquid is applied to the core. Gel blocking often leads to leakage from the absorbent article well before all of the absorbent material in the core is fully saturated.

Despite the incidence of gel blocking, superabsorbent materials are commonly incorporated into absorbent cores because they absorb and retain large quantities of liquid, even under load. However, in order for superabsorbent materials to function, and avoid premature leakage, the liquid being absorbed in the absorbent structure must be transported to unsaturated superabsorbent material. In other words, the superabsorbent material must be placed in a position to be contacted by liquid. Furthermore, as the superabsorbent material absorbs the liquid it must be allowed to swell. If the superabsorbent material is prevented from swelling, it will cease absorbing liquids.

Adequate absorbency of liquid by the absorbent core at the point of initial liquid contact and rapid distribution of liquid away from this point is necessary to ensure that the absorbent core has sufficient capacity to absorb subsequently deposited liquids. Previously known absorbent cores have thus attempted to absorb quickly and distribute large quantities of liquids throughout the absorbent core while minimizing gel blocking during absorption of multiple doses of liquid.

In general, some of the important performance attributes of an absorbent core of a diaper (or any other absorbent garment) are functional capacity, rate of absorption, core stability in use, type of SAP, ratio of fibrous material to SAP, the type and basis weight of glue or tackifying agent used to adhere the SAP to the fibrous material or tissue wrapping, and the basis weight of the core. Absorption under load or AUL is a good measure of functional capacity and the rate at which that absorption occurs. Without wishing to be bound by theory, AUL is believed to be a function of both SAP basis weight (mass per unit area) and the composition of SAP used in the composite. Increasing the basis weight decreases the performance/cost ratio of the absorbent core, making them uneconomical. Also, increased basis weights tend to affect the fit and comfort of the garment, as well as impacting the packaging and shipping costs.

It is known to provide absorbent multi-layer cores comprised of, for example, an upper layer, a lower layer and a central absorbent layer containing from 50% to 95% by weight SAP. U.S. Pat. No. 6,068,620, discloses that the upper and lower layers are comprised of tissue, airlaid fluff pulp or synthetic non-woven fibrous layers. The upper and lower layers are said to assist in maintaining the integrity of the core, the layered arrangement is said to minimize gel blocking, and the multi-layer absorbent composite can be folded in various configurations. It also is known to provide a composite absorbent structure having a wicking layer bonded to the absorbent layer with a bonding agent such that the absorbent structure has a Contact Intimacy Ratio. U.S. Pat. No. 6,239,565 discloses an absorbent composite having a wicking layer that has a vertical wicking flux value, an absorbent liquid retention layer, and a bonding agent.

It is also known to provide an absorbent core having a multiple layers comprising a mixture of fiber and superabsorbent. U.S. Pat. No. 5,728,082 discloses an absorbent body having a first layer of a mixture of fluff and a first superabsorbent, and a second layer having a second superabsorbent having a higher liquid absorbency than the first superabsorbent. U.S. Pat. No. 6,020,536 discloses an absorbent body that includes at least two mutually different cellulose fluffs disposed in two absorbent layers that may also contain superabsorbent materials. U.S. Pat. No. 5,741,241 discloses an absorbent body having a first fiber-based absorbent layer comprised of mixture of cellulose fluff pulp and SAP, a second fiber-based absorbent layer comprised of layers of cellulose fluff pulp and SAP.

There is however still a need for a core that improves liquid handling and cost-efficiencies as well as permitting a more environmentally friendly alternative to existing products on the market whilst achieving excellent performance in both absorption and rewet.

### SUMMARY

In a first aspect the disclosure relates to an absorbent article comprising: a liquid permeable topsheet, a liquid impermeable backsheet, and an absorbent core positioned between said topsheet and backsheet, wherein the absorbent core comprises an absorbent material, said absorbent material comprising a mixture, or blend, of cellulose fibers and superabsorbent polymers, wherein the absorbent core comprises at least a top layer (L1) and a bottom layer (L2) wherein the bottom layer (L2) is positioned between the top layer (L1) and the backsheet, and wherein said absorbent material is contained within at least one core wrap substrate enclosing said absorbent material therein, wherein the at least top layer (L1) and a bottom layer (L2) are directly stacked one on top of the other to form a contact zone (Zc) where the top layer (L1) directly adjoins to the bottom layer (L2), and wherein the top layer (L1) comprises one or more first superabsorbent polymer grades (SAP1) and the bottom layer (L2) comprises one or more second superabsorbent polymer grades (SAP2), wherein the first superabsorbent polymer grades (SAP1) have an AUL that is greater than the AUL of second superabsorbent polymer grades (SAP2), and wherein the first superabsorbent polymer grades (SAP1) have an AUL, as measured according to the test method herein, of greater than 15 g/g.

### BRIEF DESCRIPTION OF THE FIGURES

**Fig. 1A-C** Illustrates absorbent articles according to embodiments according to the present disclosure.
**Fig. 2** Illustrates a cross-section of absorbent articles according to embodiments according to the present disclosure.
**Fig. 3A-C** Illustrates absorbent articles according to embodiments according to the present disclosure.
**Fig. 4** Illustrates an exemplary process for the production of high-AUL Bio-SAP.
**Fig. 5** Illustrates results according to Example 1 herein.
**Fig. 6** Illustrates results according to Example 2 herein.
**Fig. 7** Illustrates an absorbent article according to embodiments according to the present disclosure.
**Fig. 8** Illustrates an absorbent article according to embodiments according to the present disclosure.

### DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed disclosure. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

"Laminate" refers to elements being attached together in a layered arrangement.

The term "spunbond fibers (or layer(s) or nonwovens)" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated to form laminate layers, for example spunbond-meltblown-meltblown-spunbond (SMMS), or spunbond-meltblown (SM) etc. The recitation of numerical ranges by endpoints includes all numbers and fractions subsumed within that range, as well as the recited endpoints unless otherwise stated.

"Carded web (or layer(s) or nonwoven)" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement. Carded thermobonded nonwoven thus refers to a carded nonwoven wherein the bonding is achieved by use of heat.

The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

"Adhesive" typically means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

As used herein, the term "transverse" or "lateral" refers to a line, axis, or direction which lies within the plane of the absorbent article and is generally perpendicular to the longitudinal direction.

"Thickness or caliper" herein are used interchangeably, and refer to the caliper typically measured as follows: at 0.5 kPa and at least five measurements are averaged. A typical testing device is a Thwing Albert ProGage system. The diameter of the foot is between 50 mm to 60 mm. The dwell time is 2 seconds for each measurement. The sample is to be stored at 23 + 2°C and at 50 + 2% relative humidity for 24 hours with no compression, then subjected to the fabric thickness measurement. The preference is to make measurements on the base substrate before modification, however, if this material is not available an alternative method can be used. For a structured substrate, the thickness of the first regions in between the second regions (displaced fiber regions) can be determined by using a electronic thickness gauge (for instance available from McMaster-Carr catalog as Mitutoyo No 547- 500). These electronic thickness gauges can have the tips changed to measure very small areas. For example, a blade shaped tip can be used that is 6.6mm long and 1 mm wide. Flat round tips can also be inserted that measure area down below 1.5mm in diameter. For measuring on the structured substrate, these tips are to be inserted between the structured regions to measure the as-produced fabric thickness. The pressure used in the measurement technique cannot be carefully controlled using this technique, with the applied pressure being generally higher than 0.5kPa.

"Dry-state" refers to the condition in which an absorbent article has not yet been saturated with exudates and/or liquid.

"Wet-state" refers to the condition in which an absorbent article has been saturated with exudates and/or liquid. Typically wherein at least 30ml, preferably at least 40ml, even more preferably at least 50ml, most preferably from 60ml to 800ml, of exudate and/or liquid are contained in the absorbent article.

As used herein, the term "cellulosic" or "cellulose" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

By "substantially", it is meant at least the majority of the structure referred to.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "consisting essentially of' does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

As used herein, the "body-facing" or "bodyside" or "skin-facing" surface means that surface of the article or component which is intended to be disposed toward or placed adjacent to the body (e.g. the face) of the wearer during ordinary use, while the "outward", "outward-facing" surface is on the opposite side, and is intended to be disposed to face away from the wearer's body during ordinary use.

"Spunlaced" as used herein refers to nonwoven fabrics or materials that are made by hydroentangling webs of fibers (and/or fibers) with high energy water jets for example as basically described in Evans et al. US Patent No. 3,485,706. The webs may be made of a variety of fibers such as polyester, rayon, cellulose (cotton and wood pulp), acrylic, and other fibers as well as some blends of fibers. The fabrics may be further modified to include antistatic and antimicrobial properties, etc. by incorporation of appropriate additive materials into the fiber or fiber webs.

"Wetlaid" as used herein means nonwovens obtained bya process similar to paper manufacturing. The difference lies in the amount of synthetic fibres present in a wetlaid nonwoven. A dilute slurry of water and fibres is deposited on a moving wire screen, where the water is drained and the fibres form a web. The web is further dewatered by pressing between rollers and dried. Impregnation with binders is often included in a later stage of the process.

"Airlaid" as used herein means a process wherein fibres, which are typcially relatively short, are fed into a forming head by an airstream. The forming head assures a homogeneous mix of all fibres. By air again, a controlled part of the fibre mix leaves the forming head and is deposited on a moving belt, where a randomly oriented web is formed. Compared with carded webs, airlaid webs have a lower density, a greater softness and an absence of laminar structure.

The term "grades" used in "superabsorbent polymer grades" typically refers to the chemichal properties e.g. monomer and charge ratio; and physicochemical properties e.g. network structure, degree of cross-linking, post treatment and particle size distribution, of the superabsorbent polymer. Typically, these properties (or grades) result in unique performance parameters such as AUL, absorbption speed (or Vortex), CRC and the like.

Embodiments of the articles and processes according to the disclosure will now be described. It is understood that technical features described in one or more embodiments maybe combined with one or more other embodiments without departing from the intention of the disclosure and without generalization therefrom.

### THE ABSORBENT ARTICLE

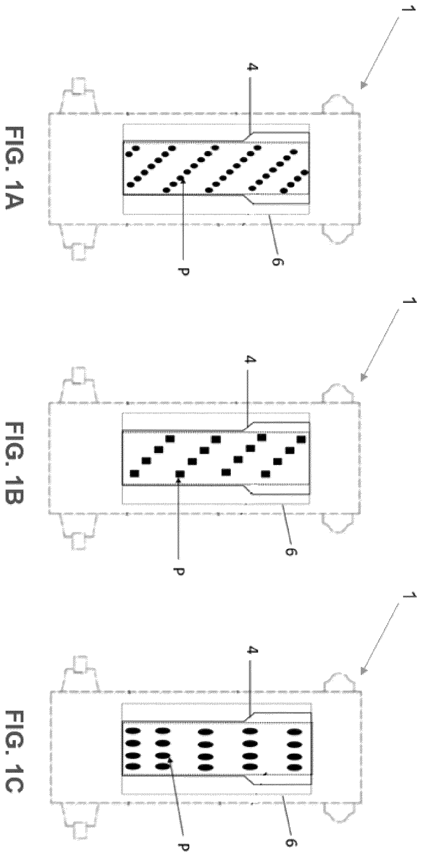

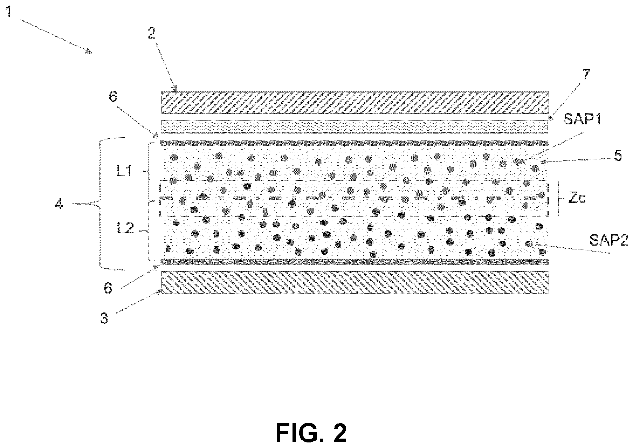

As exemplified in Fig. 1 and 2, absorbent articles herein comprise: a liquid permeable topsheet (2), a liquid impermeable backsheet (3), and an absorbent core (4) positioned between said topsheet (2) and backsheet (3), wherein the absorbent core (4) comprises an absorbent material (5), said absorbent material comprising a mixture, or blend, of cellulose fibers and superabsorbent polymers, wherein the absorbent core (4) comprises at least a top layer (L1) and a bottom layer (L2) wherein the bottom layer (L2) is positioned between the top layer (L1) and the backsheet (3), and wherein said absorbent material is contained within at least one core wrap substrate (6) enclosing said absorbent material therein. The core wrap substrate (6) may be a single sheet that is folded onto itself to contain the absorbent material layers therein or may comprise two distinct sheets that are bonded to each other at least at the peripheral edges thereof to sandwich the absorbent material therein. Several core wrap constructions are possible such as G-fold, C-fold or sandwich wraps. The core wrap substrates herein are typically composed of a nonwoven layer or nonwoven laminates comprising spunbond nonwoven, meltblown nonwoven and combinations thereof or tissue. Typically the basis weight of the core wrap substrate is from 5 gsm to 50 gsm, preferably from 6 gsm to 35 gsm, more preferably from 8 gsm to 30 gsm.

The absorbent cores as used herein comprise the at least one top (or upper) layer (L1) and bottom layer (L2) that are directly stacked one on top of the other to form a contact zone (Zc) where the top layer (L1) directly adjoins to the bottom layer (L2). It is to be understood herein that more than two layers are possible and may be stacked one on top of the other provided that the uppermost layer is in the form of the top layer herein, all other layers may be in the form of bottom layers as described herein. Preferably, the core is free of any additional substrates separating the top and bottom core layers thus allowing some blending of SAP1 and SAP2 at the contact zone which promotes optimal fluid distribution and reduced material usage.

The top layer (L1) of the absorbent core comprises one or more first superabsorbent polymer grades (SAP1) and the bottom layer (L2) comprises one or more second superabsorbent polymer grades (SAP2), wherein the first superabsorbent polymer grades (SAP1) have an AUL that is greater than the AUL of second superabsorbent polymer grades (SAP2), and wherein the first superabsorbent polymer grades (SAP1) have an AUL, as measured according to the test method herein, of greater than 15 g/g. Advantageously, particularly in absence of intermediate nonwoven or bulky layers between the core layers, having such a higher AUL SAP on the top layer of the core limits rewet by providing resistance to squeeze-out of liquid upon application of forces whilst at the same time limiting the effects of gel-blocking. The lower layer may then have fast absorbing SAP that generally comes with poorer/lower AULs in order to promote fast absorption speeds and aid the draining effect mechanism supported by the cellulose fibers acting as liquid conduits towards the dispersed SAP.

In an embodiment, the cellulose fibers are comprised at a level of at least 20%wt, preferably from 25%wt to 40%wt, even more preferably from 28%wt to 37%wt, by total weight of the absorbent material. Advantageously, cellulose fibers in these amounts allow for fast distribution of liquid across the core surface but if the amount is too high leads to poor rewet performance and/or excessive thickness and bulkiness of the core which reduces comfort of wear.

In an embodiment, and in particular preferably in further combination with the previous embodiment describing the cellulose fiber content, the SAP Ratio (SAP1/SAP2) is from 0.3 to 3, preferably from 0.5 to less than 3, even more preferably from 0.7 to 2.5, even more preferably from 1 to 2, even more preferably from greater than 1 to less than 2; and/or wherein SAP1 is comprised at a level of from more than 25%wt to 75%wt, preferably from 30%wt to less than 75%wt, even more preferably from 35%wt to 70%wt, even more preferably from 40%wt to 65%wt, even more preferably from 41% to 64%, by total weight of the superabsorbent polymers (SAP1+SAP2). Advantageously, the SAP1 ratio and/or content has been found to synergistically work with the cellulose fibers/fluff content to limit sponge-like effects and/or squeeze-out of liquid following application of a load or force.

In an alternative, or complementary, embodiment the SAP Ratio (SAP1/SAP2) is less than 1, preferably from 0.2 to 0.9, more preferably from 0.3 to 0.85, even more preferably 0.4 to 0.8; and/or wherein SAP1 is comprised at a level of from more than 10%wt to 50%wt, preferably from 15%wt to less than 50%wt, even more preferably from 20%wt to 45%wt, even more preferably from 25%wt to 40%wt, even more preferably from 30%wt to 35%wt, by total weight of the superabsorbent polymer (SAP1+SAP2).

In a highly preferred embodiment, the top layer (L1) has a first ratio (R1) of SAP1 to cellulose fibers (i.e. SAP1 weight/cellulose fibers weight) and the bottom layer (L2) has a second ratio (R2) of SAP2 to cellulose fibers (i.e. SAP2 weight/cellulose fibers weight), wherein R2 is less than R1, preferably R2 is less than 0.95R1, preferably R2 is from 0.2R1 to 0.93R1, preferably R2 is from 0.3R1 to 0.9R1, preferably R2 is from 0.4R1 to 0.85R1. Advantageously, having more cellulose fibers in the layer of the core that contains high absorption speed SAPs (typically consequentially having lower AUL) synergistically cooperates with layers having less cellulose fibers and containing higher AUL SAP (typically consequentially having a lower absorption speed) to provide fast liquid distribution that limits gel blocking effects and reduced absorbency efficiencies of the core, in particular when the bottom layer (L2) comprises a lower AUL and higher absorption speed SAP2 compared to SAP1 contained in the top layer, improved perceived dryness and rewet performance is achieved whilst negating drawbacks to speed of acquisition through the overall core.

Alternatively, R2 is greater than R1, preferably R2 is greater than 1.1R1, preferably from 1.2R1 to 2.5R1, more preferably from 1.3R1 to 2.0R1. Advantageously, this allows liquid to quickly be distributed from areas containing higher AUL SAPs to areas of the core containing lower AUL SAPs. In this embodiment, it is preferable to incorporate the use of an acquisition distribution layer as described in embodiments herein in order to negate potential rewet drawbacks generated.

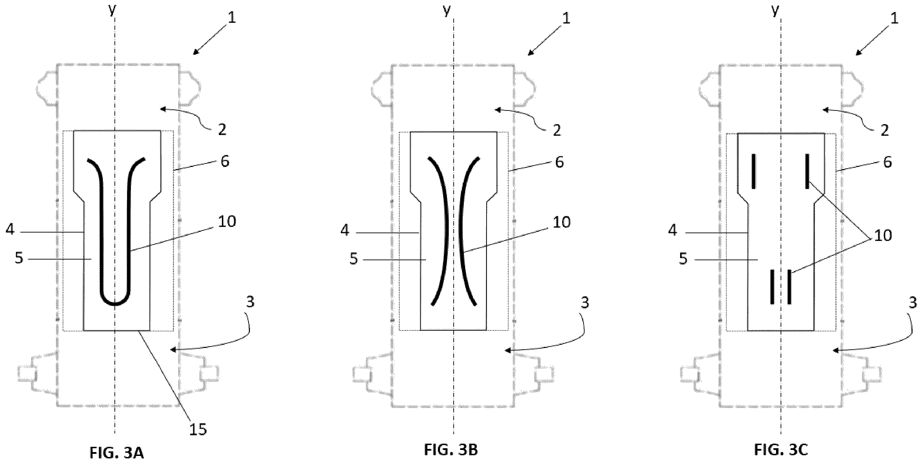

In a highly preferred embodiment, the core wrap substrate (6) comprises an upper layer and a lower layer and the absorbent material is sandwiched therebetween with the upper layer positioned at a skin-facing (or body-facing) side of the absorbent material and the lower layer positioned at an opposite garment facing side of said absorbent material. The upper and lower layers are preferably joined together at one or more attachment areas positioned inboard of a perimeter of the absorbent core such to form one or more channels (10) substantially free of absorbent material. The channel(s) typically have a length extending in the longest dimension of the core that is from 10% to 95%, preferably from 15% to 90%, even more preferably from 20% to 85%, of the length of said longest dimension of the core. Exemplary channel geometries are illustrated in Fig. 3. The upper layers of the core wrap may be joined together at the attachment areas via one or more adhesives and/or one or more mechanical bonds (such as ultrasonic bonding, pressure bonding, thermal bonding, and combinations thereof). Advantageously, such channel structures further allow to reduce the overall amount of absorbent material used thus improving cost efficiencies and further improves liquid distribution speed across the core that in turn allows for further SAP combinations as will be discussed herein.

The absorbent articles herein are preferably diapers or pants for babies or adults. In case of diapers preferred further components used in the art include: elastic ears or side panels for the fastening thereof, fastening systems comprising hook and loop and/or adhesive; leg cuffs; transversal front and back barriers or cuffs; acquisition distribution layers, wetness indicators etc. In case of pants preferred are 3-piece pants wherein front and back elasticized belts are joined via an absorbent insert comprising the topsheet, backsheet and absorbent core therebetween. The elasticized belts typically comprise a plurality of elastic strands or unitary elastic film. Further components in pants may be similar to those found in diapers such as leg cuffs; transversal front and back barriers or cuffs; acquisition distribution layers, wetness indicators etc.

In a highly preferred embodiment, the absorbent article herein further comprises an acquisition distribution layer (7) positioned between the topsheet (2) and the core wrap substrate (6), preferably an upper layer therof, and wherein the majority (typically being more than 50%, preferably more than 60%, even more preferably more than 70%, even more preferably more than 80%, even more preferably more than 80%, of the surface area of said acquisition distribution layer taken from a planar view formed by the longitudinal axis y and a transverse axis perpendicular thereto) of the surface of said acquisition distribution layer is in direct contact at least with said core wrap substrate; and wherein the acquisition distribution layer comprises, preferably consists of, a nonwoven selected from the group consisting of spunbond nonwoven, meltblown nonwoven, carded thermobonded nonwoven, and combinations thereof. Preferably, the acquisition distribution layer comprises, preferably consists of, synthetic fibers, wherein said synthetic fibers are comprised at a level of greater than 50%wt, preferably greater than 80%wt, by weight of said acquisition distribution layer, and wherein said acquisition distribution layer has a basis weight of from 10 to 50 g/m², preferably from 15 to 45 g/m², even more preferably from 20 to 40 g/m², even more preferably from 21 to 35 g/m². Surprisingly, whilst the industry has moved to the wide implementation of airlaid or other high bulky nonwoven layers for improved liquid distribution, the inventors have surprisingly found that such bulky nonwovens result in significant sponge-like effects undesirably impacting rewet performance, and hence wetness perception by the wearer. The use of such nonwovens in combination with top layers L1 as described herein synergistically reduce rewet perception as well as overall cost. The use of the specific nonwovens identified above allows to reduce rewet and overall cost of the product, and especially (but not necessarily) with the presence of the spot-bonds described herein in the absorbent core creates a network that on top of improving core stability further allows to maintain the appropriate liquid distribution properties positively impacting acquisition time with no longer necessitating bulky nonwovens.

Preferably, the core wrap substrate (6) comprises upper and lower layers that are joined together by one or more adhesives, and/or by one or more mechanical bonds selected from the group consisting of ultrasonic bonds, thermal bonds, pressure bonds, and combinations thereof. Typically the upper and lower core wrap layers are, directly or indirectly, joined together at one or more positions inboard of the perimeter of the absorbent core and may be distinct to (or disconnected or inboardly spaced from) said perimeter; or come into contact with said perimeter.

As exemplified in Fig. 1, the upper layer of the core wrap (6) is preferably joined to the bottom layer of the core wrap (6) at one or more bonding points (P) positioned inboard of a perimeter of the absorbent core (4), and preferably wherein said bonding points have an aspect ratio of less than 3, preferably of from 1 to 2, and more preferably having a shape selected from the group consisting of circular, elliptical, line-form, star-shaped, polygonal, and combinations thereof. By the term "aspect ratio" as used hereinabove it is intended the ratio of the longest dimension to the shortest dimension of said bonding points (i.e. longest length / shortest length). In addition to the network effects described above, it further optimizes core integrity of the multi-layer core construct.

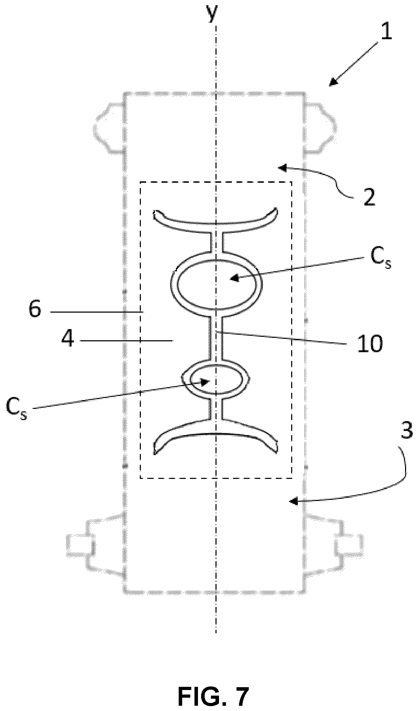

In other embodiments, as exemplified in Fig. 3A-C and/or Fig. 7, the upper and lower core wrap layers are joined together at one or more attachment zones to form one or more channels (10), preferably a single channel, substantially free of absorbent material. Advantageously such channel structures aid fluid distribution along and/or across the core. Preferably, the channel(s) described herein (i.e. any of the channel embodiments described herein) do not extend to the edges and/or perimeter of the absorbent core such that typically said channel(s) are circumscribed by absorbent material. Advantageously channel(s) that have terminal edges at a position inboard of the edges/perimeter of the absorbent core may aid to further limit risk of leakage as well as may further aid migration of the SAPs during swelling when wetted and hence accommodate for better volume expansion rates that permit overall optimal absorption efficiency.

In an embodiment, the at least a top layer (L1) and a bottom layer (L2) are directly in contact with each other so that no further material such as adhesive, nonwoven layers and/or tissue is interposed therebetween. Advantageously this may drive absorption efficiencies by limiting the amount of hydrophobic material or materials that would slow down liquid especially when using the SAP1 and SAP2 arrangement herein and work in synergy with the channel(s) described herein to provide further liquid management performance.

As exemplified in Fig. 7, preferably the channel(s) have a shape forming at least one, preferably at least two, preferably from 2 to less than 10, even more preferably from 2 to 6, even more preferably from 2 to 5, typically only two, clusters (Cₛ) of absorbent material circumscribed by said attachment zones and preferably wherein the clusters (Cₛ) are spaced apart along a dimension parallel to the longitudinal axis (y), and even more preferably wherein the clusters (Cₛ) are connected by one or more attachment zones bridging between said clusters and extending substantially along said longitudinal axis (y).

Advantageously, such clusters improve core stability of the multi-layer/multi-SAP grade cores especially upon wetting and especially in absence of any further layer (e.g. a further nonwoven or tissue) separating the two or more absorbent core layers leading to an optimal cost-effective solution that may avoid the incorporation of such further layers. However, having too many such clusters may result in a stiffening of the absorbent core upon saturation and swelling (typically as a result of wetting) of the absorbent material the optional upper limit of such clusters ensures to limit such effects for improved further comfort.

In a preferred embodiment, each cluster (Cₛ) has an area (on a plane corresponding to that formed by the longitudinal axis y and the width of the absorbent article perpendicular to said longitudinal axis y, i.e. as viewed from a planar view as exemplarily illustrated in Fig. 7) that is from 5% to 35%, preferably from 8% to 30%, more preferably from 10% to 25%, even more preferably from 12% to 20%, of the area of the absorbent core (on said same plane). Advantageously this also contributes to limit stiffness upon saturation. Typically said area being the area formed inboard of the attachment zones circumscribing said clusters of absorbent material and may be calculated by tracing the innermost edge of said attachment zones to determine the perimeter thereof and then calculate the respective area therefrom. Alternatively, image analysis may be used to determine the % areas, for example by taking a top view image (in a planar view as shown in Fig. 7); cropping the image accordingly so that the image sizing matches the core dimensions; convert the image into back&white; assign black pixels to the image in the cluster areas inboard of the attachment zones; assign white pixels to remaining areas of the image; determine the % area by dividing the total black pixels in the image by the total sum of black and white pixels in the image.

In a preferred embodiment, at least two clusters (Cₛ) of the one or more clusters have an area (on a plane corresponding to that formed by the longitudinal axis y and the width of the absorbent article perpendicular to said longitudinal axis y, i.e. as viewed from a planar view as exemplarily illustrated in Fig. 7) that is different in that said area of a first cluster is greater than said area of a second cluster, preferably wherein the first cluster is positioned closer to a front end of the diaper compared to the second cluster (as exemplarily shown in Fig. 7).

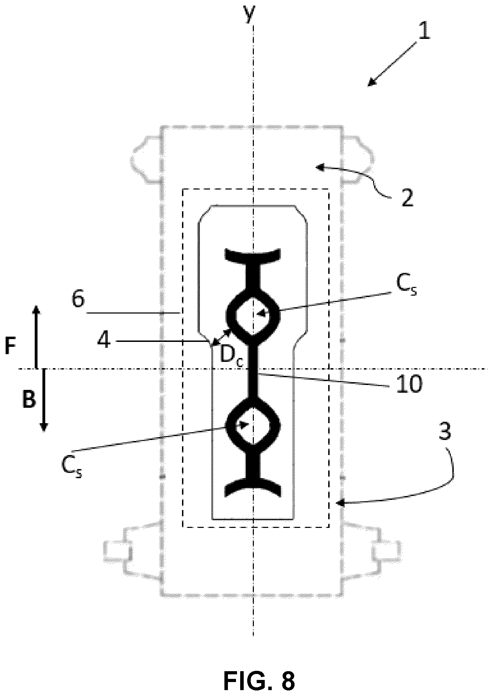

Preferably, as exemplified in Fig. 8, the shortest distance (Dc) between an absorbent core perimeter edge and the closest position on the channel (typically at a position in the front half F and generally distal from the back half B of the article) is greater than the minimum width of the channel, preferably at least 1.5 times the minimum width of the channel, even more preferably at least 2.0 times minimum width of the channel. Preferably the shortest distance (Dc) is from 10mm to 50mm, preferably from 15mm to 45mm, even more preferably from 20mm to 40mm, even more preferably from 25mm to 35mm. Advantageously this limits risk of leakage.

In an embodiment, as exemplified in Fig. 8, at least two clusters (Cₛ) of the one or more clusters have an area (on a plane corresponding to that formed by the longitudinal axis y and the width of the absorbent article perpendicular to said longitudinal axis y, i.e. as viewed from a planar view as exemplarily illustrated in Fig. 8) that is substantially equal.

In a highly preferred embodiment, the contact zone (Zc) comprises a blend of first superabsorbent polymer grades (SAP1) and second superabsorbent polymer grades (SAP2), preferably that are intermixed typically such that a layered absorbent core is formed having: a top layer (L1) wherein the superabsorbent polymer consists of said first superabsorbent polymer grades (SAP1); a bottom layer (L2) wherein the superabsorbent polymer consists of said second superabsorbent polymer grades (SAP2); and a contact zone (Zc) between said top and bottom layers (L1, L2) comprising a mixture of first superabsorbent polymer grades (SAP1) and second superabsorbent polymer grades (SAP2). Advantageously this allows for a core stabilization layer to be formed in the contact zone Zc that provides for core integrity improvement without the inclusion of additional intermediate layers such as nonwoven or bulky layers between absorbent material layers. Preferably, said contact zone Zc is arranged to form a substantially reticular structure upon liquid saturation and swelling of SAP1, SAP2 and the cellulose fibers. Without wishing to be bound by theory the contact zone Zc benefitting from a mix of SAP1 and SAP2 grades and cellulose fibers allows to attain a stiffening structure (typically in the thickness direction) upon wetting and formation of a sort of reticular structure that limits migration of absorbent material from the top layer L1 to the bottom layer L2 thus improving core stability and integrity without addition of other materials and structural features that may add cost and/or be a source of negative environmental impact. Also, this allows the use of SAP grades that by themselves normally would negatively influence the performance of the core such as many Bio-SAPs which, although environmentally friendly, do still suffer performance over classic non-Bio-SAPs.

In a preferred embodiment, the contact zone (Zc) has a first thickness, the top layer L1 has a second thickness, and the bottom layer has a third thickness, each running parallel to the an absorbent core thickness (as exemplified in Fig. 2) and wherein the sum of the first, second and third thicknesses are equal to the absorbent core thickness. Although reference is made to top and bottom layers L1 and L2, it is understood herein that there may be further layers such as cellulose fiber dusting layers adjacent the top and bottom layers L1 and L2 positioned at a contact surface being opposite the contact zone. The first thickness is less than the second and third thicknesses. Preferably the second thickness is substantially greater or equal to the third thickness. Most preferably the first thickness is from 5% to 70%, preferably from 10% to 60%, more preferably from 15% to 50%, even more preferably from 20% to 40%, of the second thickness. Thickness measurements are done using known methods such as by wetting an absorbent core of a diaper with 250 mL of saline solution (e.g. saline with 0.9 wt % NaCl), with or without a coloring dye e.g. blue, applying a waiting time of 30 seconds (at ambient/room conditions), cutting the diaper along the mid transversal centerline of the absorbent core running along the width thereof, and taking measurements with a caliper. Alternatively, the cross-sections may be put under a microscope or SEM to identify the SAP grades respectively and thickness measurements run thereon. Alternatively, it may be ensured by machine settings during production in particular the airflow speed in the absorbent material deposition step and may thus be requested by the manufacturer.

### THE SUPERABSORBENT MATERIAL

Superabsorbent polymers, superabsorbent polymers particles, or SAPs refer to water-swellable, water-insoluble organic or inorganic materials capable of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present disclosure in an amount up to about 90% by weight.

Absorbent cores comprised in absorbent articles of the present disclosure comprise specific SAP grades and/or types in specific layers of the multi-layer core construct.

Typically, the superabsorbent polymers referred to herein are in the form of particles or granules.

In an embodiment, the second superabsorbent polymer grades (SAP2) herein have an AUL of less than 15 g/g, preferably from 5 g/g to 14 g/g, even more preferably from 8 g/g to 12 g/g. The second superabsorbent polymer grades (SAP2) typically has an absorbption speed, according to the test method herein, of less than 45 seconds, preferably from 5 seconds to 40 seconds, even more preferably from 15 seconds to 35 seconds. Advantageously this allows for extremely fast liquid uptake though with some compromise to rewet performance and uptake under load.

Preferably, the first superabsorbent polymer grades (SAP1) have an AUL of greater than 18 g/g, preferably from 19 g/g to 55 g/g, even more preferably from 20 g/g to 50 g/g, even more preferably from 21 g/g to 45 g/g, even more preferably from 22 g/g to 35 g/g. The first superabsorbent polymer grades (SAP1) typically have an absorbption speed, according to the test method herein, of more than 50 seconds, preferably more than 55 seconds, more preferably from 60 seconds to 150 seconds, even more preferably from 70 seconds to 100 seconds. Advantageously this allows reduced rewet (indication of dryness on the skin-facing surface) because of the high AUL on the upper surface that "releases" less liquid under load/pressure e.g. weight of the baby, with compromise on absorption speed and speed of liquid uptake.

It will be evident to a person skilled in the art that the multi-layered arrangement described herein negates the individual compromises of SAP1 and SAP2 grades taken individually and that are rather arranged to work synergistically in combination for added core functionality when combined with cellulose fibers as described herein.

In an embodiment, the top layer (L1) comprises a plurality of superabsorbent polymer grades, and wherein the difference in AUL between said grades is less than 15%, preferably less than 10%, even more preferably less than 5%, more preferably from 0% to 3%, even more preferably from 0.1% to 2%. Advantageously, this allows to reduce risk of gel blocking.

In an embodiment, the bottom layer (L2) comprises a plurality of superabsorbent polymer grades, and wherein the difference in AUL between said grades is from 0% to 50%, preferably from 5% to 45%, even more preferably from 10% to 40%, more preferably from 15% to 35%, even more preferably from 16% to 33%, even more preferably from 18% to 30%. Without wishing to be bound by theory, on lower layers the impact of gel blocking is to a lesser degree and hence a broader class of SAP grades may be used impacting reduced cost, wider choice of environmentally friendly SAPs and the like.

In a highly preferred embodiment, the AUL ratio AUL_{SAP1}/AUL_{SAP2}) of the first superabsorbent polymer grades (SAP1) and second superabsorbent polymer grades (SAP2) is greater than 1.4, preferably greater than 1.5, more preferably from 1.6 to 5, even more preferably from 1.7 to 3. Advantageously, this allows to ensure the right balance of reduced rewet and fast liquid absorption on the lower layer and optimal performance under load.

Preferably the second superabsorbent polymer grades (SAP2) comprises, preferably consists of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1 herein incorporated by reference; SAPs comprising a 100% acrylic acid co-acrylamide with little to no cross link shell, and the like all generally having AUL of less than 20 g/g according to the test method herein.

Preferably, the first superabsorbent polymer grades (SAP1) are free of Low-AUL Bio-SAP. Advantageously this limits rewet drawbacks as described hereinabove in more detail.

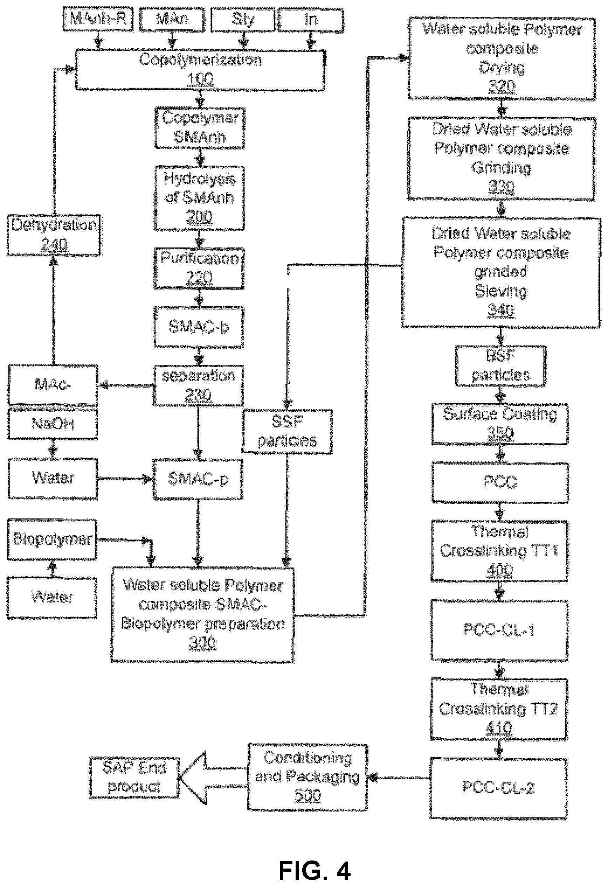

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 4. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert² (https://www.redcert.org/images/SP_RC%C2%B2_Biomass-balanced_products-V1.0.pdf ). An example may be a polyacrylic acid, sodium salt, crosslinked, partly neutralized SAP from up to 100% allocated biomass feedstock such as commercially available HySorb^{®} B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly (maleic anhydride-co- methyl vinyl ether) (Gantrez), poly (vinyl chloride-co-maleic acid) and poly [(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 4, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IOmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003- 0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 1. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white color with a value of bulk density of 0.6-0.8 g/cm3. Further, mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contain.

The processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). The purified filtrate of SMAC polymer is typically collected in an equipment as Sigma mixer type in order to be processed with biopolymers to obtain the water soluble composite polymer containing synthetic SMAC polymer and biopolymer [WSPC] (process 300).

The preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

The amount of biopolymer relative to copolymer in the composite is preferably from 4-6 % (dry basis), preferably 8-10 % (dry basis) and most preferably 12-14 % (dry basis) .

The blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30 -90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-I40°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi , which represents the end product of the manufacturing process which is the object of the present invention, i.e. the "High-AUL biodegradable superabsorbent composite polymer" referred to herein.

### AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 µm. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as W0. Then 0.900 +/- 0.005 g of water-absorbing polymer or material (particle size distribution 150 - 800 µm or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 µm in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:
AUL0.7psig/g=Wb-Wa/Wa-W0

AUL 0.3 psi and 0.5 psi are measured similarly at the appropriate lower pressure.

### Absorbption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:
1.Beaker, 100 ml.
2. Programmable magnetic stir plate, capable of providing 600 revolutions per minute.
3. Magnetic stir bar without rings, 7.9 mm × 32 mm, Teflon covered.
4. Stopwatch.
5. Balance, accurate to ± 0.01 g.
6. Saline solution, 0.9%.
7. Weighing paper.
8. Room with standard condition atmosphere: Temp = 23°C ± 1°C and Relative Humidity = 50% ± 2%.

Test procedure:
1.Measure 50 g ± 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g ± 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

### EXAMPLE 1

This example shows a representative variant for production of 10kg biodegradable SAP with high AUL for use according to embodiments of the present disclosure.

In a Sigma mixer (60 L) connected to vacuum, with a heating-cooling mantle, thermometer, and dosing funnel for liquids is inserted 2.59 kg of maleic anhydride technical grade (MAnh) and 12.07 kg recovered maleic anhydride (MAnh-R) from a previous batch. The solid mass is heated at temperature of 60°C under mixing conditions until a homogenous transparent liquid is obtained which represents melted maleic anhydride. Then, 3.17 L of styrene containing 0.2016 g of 4 -tert-butyl catechol as stabilizer and 5.04 g of benzoyl peroxide (BPO) as initiator (prepared in advance) are added in the mixer by using a dosing funnel at temperature of 5°C. After about 5 minutes from the styrene dosing, the reaction mass returns to a temperature of 60°C. After about 30 minutes at the same temperature begin the process of copolymerization, noted by increase of the temperature of the reaction mass with a speed of 0.2degree/minute.

When the mass of reaction reaches the temperature of 68°C, the reaction mass is cooled, by using a cooling agent with temperature of -8°C, after about 5 minutes the reaction mass reaches the temperature of 123°C and then begins to decrease. In this moment the cooling is stopped and when the reaction mass reaches a temperature of 100°C, a heating aid is inserted in the mixer's mantle to maintain the temperature of reaction mass at 100°C for 30 minutes. The reaction mass is then cooled to a temperature of 80°C. From this moment begins the developing of hydrolysis process of the maleic anhydride included in the polymer of SMA and also the free maleic anhydride, both being converted to maleic acid. The hydrolysis process occurs also in Sigma mixer by addition of 3.3 L of water in total, from which 1.6 L as 0.05N hydrochloric acid solution in two equal servings at 15 minutes interval and the rest of 1.7 L as deionized water which does not contain hydrochloric acid , inserted also in two servings at 60 minutes interval.

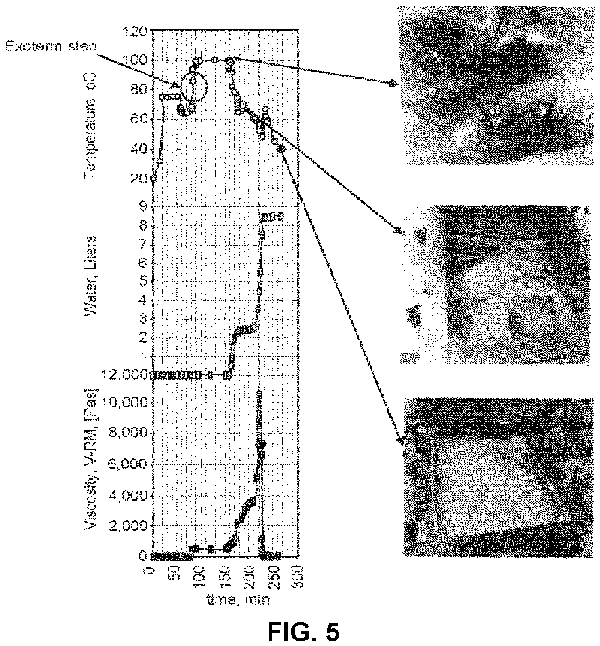

Variation in time of some properties and the aspect of the reaction mass from the moment when begins the copolymerization and to the end of the hydrolysis of the maleic anhydride free and linked to the copolymer SMAC are presented in Fig. 5.

Further the whole quantity of wet solid obtained after copolymerization and hydrolysis which represents 26.35 Kg is subdue to a process of separation of the copolymer styrene-alt maleic acid SMAC and its purification by successive extractions and filtrations using a Nuce filter with stirrer. For this purpose, over the quantity of 26.35 kg of wet reaction mass obtained in Sigma mixer is added 63 L of deionized water. The washing process occurs at temperature of 60°C for 30 minutes, then the resulted suspension is filtered under pressure of 1.1 atm. It resulted 60 L of supernatant and 28 kg filtrate. The filtrate as wet solid is subdue to a new extractions with 63 L of deionized water, the suspensions resulted is mixed at 60°C for 60 minutes. After this second filtration are obtained 31 kg filtrate and 59 L of supernatant.

Whole quantity of 119 L supernatant shall be further processed for recovery of maleic acid which it contains and then its converting in maleic anhydride necessary to the new batch of synthetic polymer. After the concentration of supernatant by reverse osmosis from 15% to 30% , further is subdue to atomization to obtain the maleic acid. In the end by thermal dehydration under vacuum (by modifying the method described in US Pat No. 4,414,398) are obtained 12.07 kg of recovered maleic anhydride (MAnh-R].

The wet solid mass of 31 kg that represents the filtrate is transferred in Sigma mixer to prepare the polymeric composite following addition of the biopolymer. This process occurs as follow: in Sigma mixer over 31 kg of wet SMAC is added 4.52. L of NaOH solution of 40% concentration, and is obtained a quantity of 35.52 kg of viscous mass that represents styrene maleic acid copolymer as sodium salt of 16.37 % concentration.

Separately in a reaction vessel with stirrer and heating -cooling mantle, 1.28L of gelatin Type A ( Bloom 150) solution of 20% concentration is prepared .

Finally in Sigma mixer is mixed at a temperature of 65°C for 2 hours 35.52 kg solution of synthetic polymer with 1.28 L of gelatin solution to which is added also 1.83 kg of small solid phase of SAP resulted from a previous batch. Further the homogenous mass from mixer is dehydrating under vacuum of 10 mbar for 5 hours when its humidity content reaches 23.4 % and the mass has a granular aspect called WSPC (water soluble polymer composite).

Instead of poly(styrene-maleic acid) copolymer, other synthetic polymers are used, for example poly(maleic anhydride-co-methyl vinyl ether) (Gantrez), poly(vinyl chloride- co-maleic acid) and poly[(maleic anhydride)-alt-(vinyl acetate).

The polymeric material WSPC that results after preparation is subdue to a supplementary drying. In this sense occurs the transport of granular mass of partial dried composite to a dryer with pre-heated air at temperature of 75-85°C as rotary type and is dried until the humidity content will be less than 7.44% .

Further the WSPC supplementary dried is grinded and sieved in several steps so in the end are obtained two types of solid phases called big solid phase BSF and small solid phase SSF (with the biggest particle under 100 microns). By grinding with conical mills are obtained 1.83 kg SSF and 8.1 kg of LSP with the granulometric distribution presented in Table 1.

The 8.1 kg of LSP are coated with 0.07 kg of PEG -200 solution during 90 minutes at temperature of 45°C.

The coated product is subdue to a thermal treatment at temperature of 110°C during 90 minutes (TT1), when is obtained the material PCC-CL-I which is let to cool until it reaches a temperature of 40 °C . Then is subdue to a second thermal treatment which occurs at temperature of 130°C for 15 minutes. After cooling and conditioning at room temperature are obtained 10kg SAP.

According to the production process presented in this example 3 batches are prepared for testing the reproducibility of the technology. The results of the technical characteristics of SAP materials result in conformity with the present disclosure are shown in Table 1.

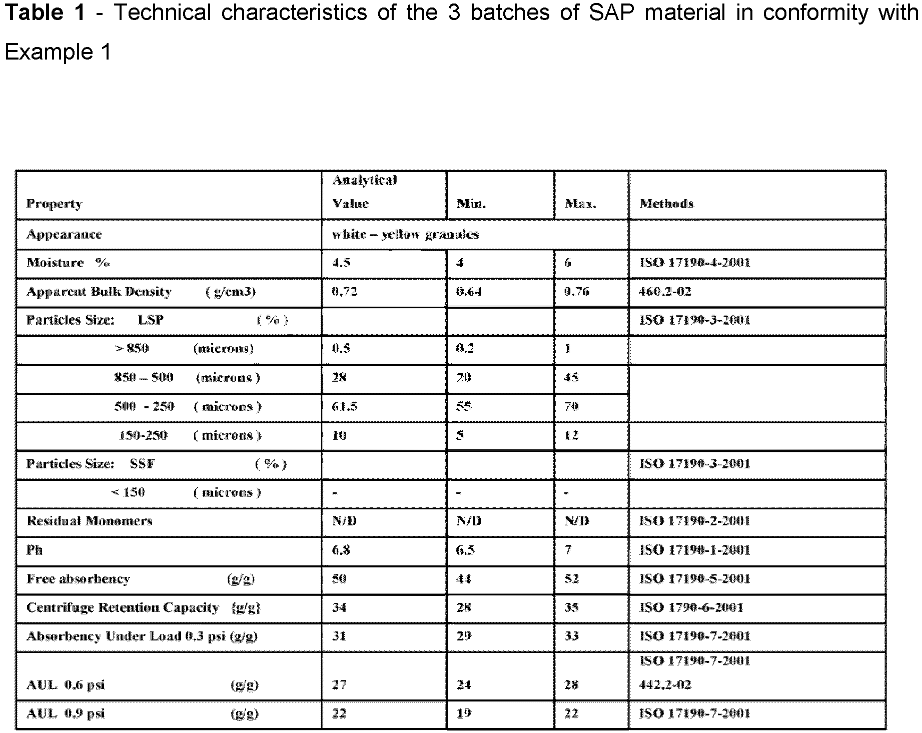

The test methods are in conformity with ISO 171901-2001

### EXAMPLE 2

Absorbent articles in this example are baby diapers. In particular all diapers tested are identical apart from the absorbent core composition that comprises different ratios of SAP1 and SAP2, and contained in a total amount of 12.3g and combined with 5g of cellulose fibers/fluff. At least 5 samples at each concentration are used in this example.

The following SAP grades are used:
SAP1 - Ekotec EK-X52A (AUL 0,7 psi 20 g/g; absorption speed/Vortex 70 sec);
SAP2 - Sumitomo SA60N Typ II, sold under the Aqua Keep brand (AUL 0,7 psi 12 g/g; absorption speed/Vortex 34 sec).

Similar samples are prepared as above, with 12.3g of SAP1 and SAP2 but this time with 7g of cellulose fibers/fluff. At least 5 samples at each concentration are used in this example.

The front and the back regions of each sample of baby diaper described above, are clamped between two spacers to allow bowl shape formation. 210 mL tap water is poured into the formed bowl-shaped cavity on the body facing side of the diaper. The liquid is allowed to be taken up by the absorbent core for 30 seconds. After the 30 seconds are elapsed the diaper is squeezed for 5 seconds. While wringing the body facing side faces toward the outside, allowing the core to twist on itself, the squeezed water is collected in a receptacle and weighed and the "squeezed water" in g is recorded for each sample tested.

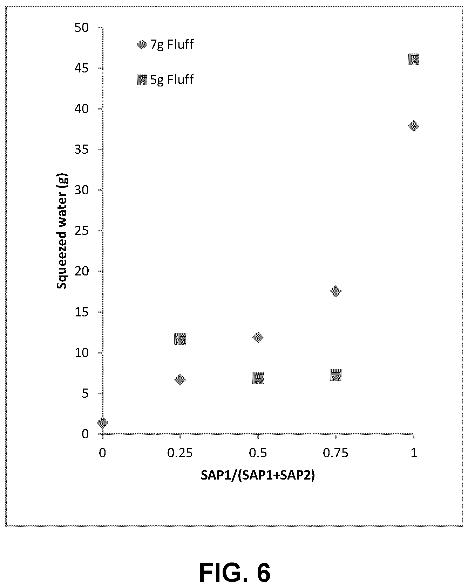

Results are reported in Fig. 6 and show the synergistic behavior between SAP1 ratio/content vs fluff content in the absorbent cores herein.

It is supposed that the present invention is not restricted to any form of realization described previously and that some modifications can be added to the presented example of fabrication without reappraisal of the appended claims.

## Claims

1. An absorbent article (1) comprising:
a liquid permeable topsheet (2),
a liquid impermeable backsheet (3), and
an absorbent core (4) positioned between said topsheet (2) and backsheet (3),
wherein the absorbent core (4) comprises an absorbent material (5), said absorbent material comprising a mixture, or blend, of cellulose fibers and superabsorbent polymers, wherein the absorbent core (4) comprises at least a top layer (L1) and a bottom layer (L2) wherein the bottom layer (L2) is positioned between the top layer (L1) and the backsheet (3), and wherein said absorbent material is contained within at least one core wrap substrate (6) enclosing said absorbent material therein, **characterized in that** the at least top layer (L1) and a bottom layer (L2) are directly stacked one on top of the other to form a contact zone (Zc) where the top layer (L1) directly adjoins to the bottom layer (L2), and wherein the top layer (L1) comprises one or more first superabsorbent polymer grades (SAP1) and the bottom layer (L2) comprises one or more second superabsorbent polymer grades (SAP2), wherein the first superabsorbent polymer grades (SAP1) have an AUL that is greater than the AUL of second superabsorbent polymer grades (SAP2), and wherein the first superabsorbent polymer grades (SAP1) have an AUL, as measured according to the test method herein, of greater than 15 g/g, and wherein the top layer (L1) has a first ratio (R1) of SAP1 to cellulose fibers and the bottom layer (L2) has a second ratio (R2) of SAP2 to cellulose fibers and **in that** R2 is less than R1.

2. An absorbent article (1) according to Claim 1 wherein the first and second superabsorbent polymers are in the form of particles or granules, fibers, and mixtures thereof.

3. An absorbent article (1) according to any of the preceding Claims wherein the second superabsorbent polymer grades (SAP2) have an AUL of less than 15 g/g, preferably from 5 g/g to 14 g/g, even more preferably from 8 g/g to 12 g/g.

4. An absorbent article (1) according to any of the preceding Claims wherein the first superabsorbent polymer grades (SAP1) have an AUL of greater than 18 g/g, preferably from 19 g/g to 55 g/g, even more preferably from 20 g/g to 50 g/g, even more preferably from 21 g/g to 45 g/g, even more preferably from 23 g/g to 35 g/g.

5. An absorbent article (1) according to any of the preceding Claims wherein the top layer (L1) comprises a plurality of superabsorbent polymer grades, and wherein the difference in AUL between said grades is less than 15%, preferably less than 10%, even more preferably less than 5%, more preferably from 0% to 3%, even more preferably from 0.1% to 2%.

6. An absorbent article (1) according to any of the preceding Claims wherein the bottom layer (L2) comprises a plurality of superabsorbent polymer grades, and wherein the difference in AUL between said grades is from 0% to 50%, preferably from 5% to 45%, even more preferably from 10% to 40%, more preferably from 15% to 35%, even more preferably from 16% to 33%, even more preferably from 18% to 30%.

7. An absorbent article (1) according to any of the preceding Claims wherein the AUL ratio AUL_{SAP1}/AUL_{SAP2}) of the first superabsorbent polymer grades (SAP1) and second superabsorbent polymer grades (SAP2) is greater than 1.4, preferably greater than 1.5, more preferably from 1.6 to 5, even more preferably from 1.7 to 3.

8. An absorbent article (1) according to any of the preceding Claims wherein the contact zone (Zc) comprises a blend of first superabsorbent polymer grades (SAP1) and second superabsorbent polymer grades (SAP2), preferably that are intermixed typically such that a layered absorbent core is formed having: a top layer (L1) wherein the superabsorbent polymer consists of said first superabsorbent polymer grades (SAP1); a bottom layer (L2) wherein the superabsorbent polymer consists of said second superabsorbent polymer grades (SAP2); and a contact zone (Zc) between said top and bottom layers (L1, L2) comprising a mixture of first superabsorbent polymer grades (SAP1) and second superabsorbent polymer grades (SAP2).

9. An absorbent article (1) according to any of the preceding Claims wherein the second superabsorbent polymer grades (SAP2) comprises, preferably consists of, Low-AUL Bio-SAP.

10. An absorbent article (1) according to any of the preceding Claims wherein the first superabsorbent polymer grades (SAP1) are free of Low-AUL Bio-SAP; and/or comprises, preferably consists of, High-AUL Bio-SAP.

11. An absorbent article according to any of the preceding Claims wherein the cellulose fibers are comprised at a level of at least 20%wt, preferably from 25%wt to 40%wt, even more preferably from 29%wt to 37%wt, by total weight of the absorbent material.

12. An absorbent article according to any of the preceding Claims wherein the core wrap substrate (6) comprises upper and lower layers that are joined together by one or more adhesives; and/or by one or more mechanical bonds selected from the group consisting of ultrasonic bonds, thermal bonds, pressure bonds, and combinations thereof; and preferably wherein said layers are joined together at one or more attachment zones to form one or more channels (10), preferably a single channel, substantially free of absorbent material, and preferably wherein the channel(s) have a shape such to form at least one, preferably at least two, typically only two, clusters (Cₛ) of absorbent material circumscribed by said attachment zones and preferably wherein the at least two clusters (Cₛ) are spaced apart along a dimension parallel to the longitudinal axis (y), and even more preferably wherein the two or more clusters (Cₛ) are connected by one or more attachment zones bridging between said clusters and extending substantially along said longitudinal axis (y).

13. An absorbent article according to Claim 12 wherein the upper layer is joined to the bottom layer at one or more bonding points (P) positioned inboard of a perimeter of the absorbent core (4), and preferably wherein said bonding points have an aspect ratio of less than 3, preferably of from 1 to 2, and more preferably having a shape selected from the group consisting of circular, elliptical, line-form, star-shaped, polygonal, and combinations thereof.

14. An absorbent article according to any of the preceding Claims further comprising an acquisition distribution layer (7) positioned between the topsheet (2) and the core wrap (6), and wherein the majority of the surface of said acquisition distribution layer is in direct contact at least with said core wrap substrate (6); and wherein the acquisition distribution layer comprises, preferably consists of, a nonwoven selected from the group consisting of spunbond nonwoven, meltblown nonwoven, carded thermobonded nonwoven, and combinations thereof.

15. An absorbent article according to any of the preceding Claims wherein the first superabsorbent polymer grades (SAP1) have an absorption speed, according to the test method herein, of more than 50 seconds, preferably more than 55 seconds, more preferably from 60 seconds to 150 seconds, even more preferably from 70 seconds to 100 seconds, and preferably wherein the second superabsorbent polymer grades (SAP2) have an absorption speed, according to the test method herein, of less than 45 seconds, preferably from 5 seconds to 40 seconds, even more preferably from 15 seconds to 35 seconds.

16. An absorbent article according to any of the preceding Claims wherein the SAP Ratio (SAP1/SAP2) is from 0.3 to 3, preferably from 0.5 to less than 3, even more preferably from 1 to 2.5, even more preferably from greater than 1 to less than 2.5; and/or wherein SAP1 is comprised at a level of from more than 25%wt to 75%wt, preferably from 30%wt to less than 75%wt, even more preferably from 35%wt to 70%wt, even more preferably from 40%wt to 65%wt, even more preferably from 50% to 64%, by total weight of the superabsorbent polymer (SAP1+SAP2).

17. An absorbent article according to any of Claims 1 to 15, wherein the SAP Ratio (SAP1/SAP2) is less than 1, preferably from 0.2 to 0.9, more preferably from 0.3 to 0.85, even more preferably 0.4 to 0.8; and/or wherein SAP1 is comprised at a level of from more than 10%wt to 50%wt, preferably from 15%wt to less than 50%wt, even more preferably from 20%wt to 45%wt, even more preferably from 25%wt to 40%wt, even more preferably from 30%wt to 35%wt, by total weight of the superabsorbent polymer (SAP1+SAP2).

18. An absorbent article according to any of the preceding Claims wherein the at least a top layer (L1) and a bottom layer (L2) each comprise an absorbent material (5), said absorbent material comprising a mixture, or blend, of cellulose fibers and superabsorbent polymers, preferably wherein said bottom layer (L2) is substantially free of the second superabsorbent polymer grades (SAP2).

19. An absorbent article according to any of the preceding Claims wherein R2 is less than 0.95R1, preferably R2 is from 0.2R1 to 0.93R1, more preferably R2 is from 0.2R1 to 0.9R1, even more preferably R2 is from 0.2R1 to 0.85R1.
